Europäisches Patentamt

European Patent Office

Office européen des brevets

(11) Publication number: **0 232 871**
**A2**

(12) # EUROPEAN PATENT APPLICATION

(21) Application number: **87101675.4**

(22) Date of filing: **06.02.87**

(51) Int. Cl.⁴: **C12P 21/00** , **C12N 5/00** , **C12N 15/00** , **G01N 33/577** , **G01N 33/574**

The applicant has filed a statement in accordance with Rule 28 (4) EPC (issue of a sample only to an expert). Accession number(s) of the deposit(s): IFO 50090

(30) Priority: **07.02.86 JP 26544/86**
**10.03.86 JP 52264/86**

(43) Date of publication of application:
**19.08.87 Bulletin 87/34**

(84) Designated Contracting States:
**AT BE CH DE FR GB IT LI NL SE**

(71) Applicant: **Yoshitomi Pharmaceutical Industries, Ltd.**
**35 Hiranomachi 3-chome Higashi-ku Osaka-shi Osaka 541(JP)**
Applicant: **Taniguchi, Masaru**
**17-12, Konakadai 3-chome Chiba-shi Chiba(JP)**

(72) Inventor: **Taniguchi, Masaru**
**17-12, Konakadai 3-chome Chiba-shi Chiba(JP)**
Inventor: **Saito, Hiroaki**
**18-9, Chishirodai-minami 1-chome Chiba-shi Chiba(JP)**
Inventor: **Yamaguchi, Yutaka**
**6-3, Tendai 4-chome Chiba-shi Chiba(JP)**
Inventor: **Hiraoka, Fumio**
**25-3, Asahigaoka 2-chome Hino-shi Tokyo(JP)**
Inventor: **Nakamura, Ikuro**
**4-1-803, Motomachi 1-chome Kiyose-shi Tokyo(JP)**

(74) Representative: **Vossius & Partner**
**Siebertstrasse 4 P.O. Box 86 07 67 D-8000 München 86(DE)**

(54) Human monoclonal antibody, hybridoma producing the same and its use.

(57) Described is a human monoclonal antibody capable of reacting with human lung cancer cells and human-mouse hybridoma which can stably produce the antibody. The human monoclonal antibody can be widely utilized as an agent for investigations on lung cancer, or as a diagnostic agent in, for example, serological diagnosis or the radioimaging of tumors using the labeled antibody, or a therapeutic agent by, for example, administering the antibody having an anti-tumor effect in vivo, etc.

Fig. 1

1a

# HUMAN MONOCLONAL ANTIBODY, HYBRIDOMA PRODUCING THE SAME AND ITS USE

## Background of the Invention

By establishment of the hybridoma method by Köhler and Milstein (1975), it has been possible to fuse antibody-producing B cells with myeloma cells to produce continuously growing B cell hybridoma which produce monoclonal antibodies (Köhler et al., Nature, 256, 495, 1975). Using this method, many monoclonal antibodies which react with desired antigens have been produced. Human monoclonal antibodies have also been produced by transformation of lymphocytes using EBV (Epstein-Barr virus), etc., in addition to cell fusion between human lymphocytes and mouse myeloma cells or human myeloma cells.

Monoclonal antibodies to malignant tumor cells are utilized for basic investigations such as analysis of tumor antigens. In addition, clinical applications including serological diagnosis, radioimaging of tumors using labeled antibodies, administration of antibodies with anti-tumor effects in vivo, etc. have been attempted.

At present, cancer therapies combine surgical therapy, chemotherapy, radiotherapy and immunotherapy. These therapies are also applied to lung cancer but relapse due to local and remote metastasis is not unusual so that lung cancer has an unfavorable prognosis among cancers. Under such circumstances, it is indispensable to produce monoclonal antibodies to lung cancer, from an aspect of new specific immunotherapy added to the treatments of lung cancer. Mention may be made of monoclonal antibodies capable of reacting with human lung cancer in, for example, Published Unexamined Japanese Patent Application Nos. 60-58926 and 60-199830, EP-156578-A and EP-155172-A.

However, anti-lung cancer monoclonal antibodies currently produced are mouse monoclonal antibodies produced by hybridomas obtained by cell fusion of antibody-producing cell of a mouse immunized by human lung cancer cell lines with mouse myeloma cells. These mouse monoclonal antibodies are foreign to the human so that they are unsuited for direct administration in the living body. It is therefore desirable to produce human monoclonal antibodies having high reactivity.

Among methods for producing human monoclonal antibodies currently used, the human-human hybridoma method involves disadvantages that human myeloma cells having a fusion efficiency comparable to mouse myeloma cells have not been established yet, stable antibody-producing clones are obtained only with difficulty even by the EBV method, and handling and separation of virus are difficult, etc. Further the production of monoclonal antibodies to human lung cancer cells by the human-mouse heterohybridoma method has not been successful on a full scale.

## Summary of the Invention

As a result of extensive investigations, the present inventors have produced human monoclonal antibody capable of strongly reacting with human lung squamous and adenocarcinomas and a human-mouse hybridoma which grows while stably producing the antibody.

The present invention, therefore relates to a human-mouse hybridoma which is obtained by cell fusion of antibody-producing human lymphocytes of lung cancer patients and mouse myeloma cells and to the human monoclonal antibody produced by this human-mouse hybridoma.

## Brief Description of the Drawings

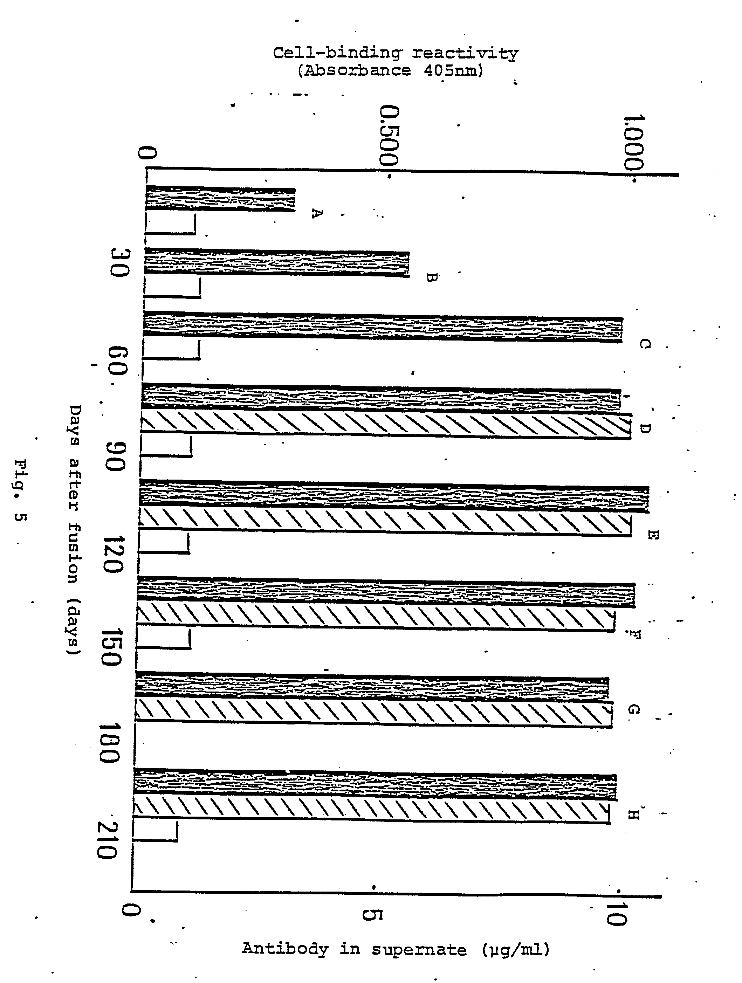

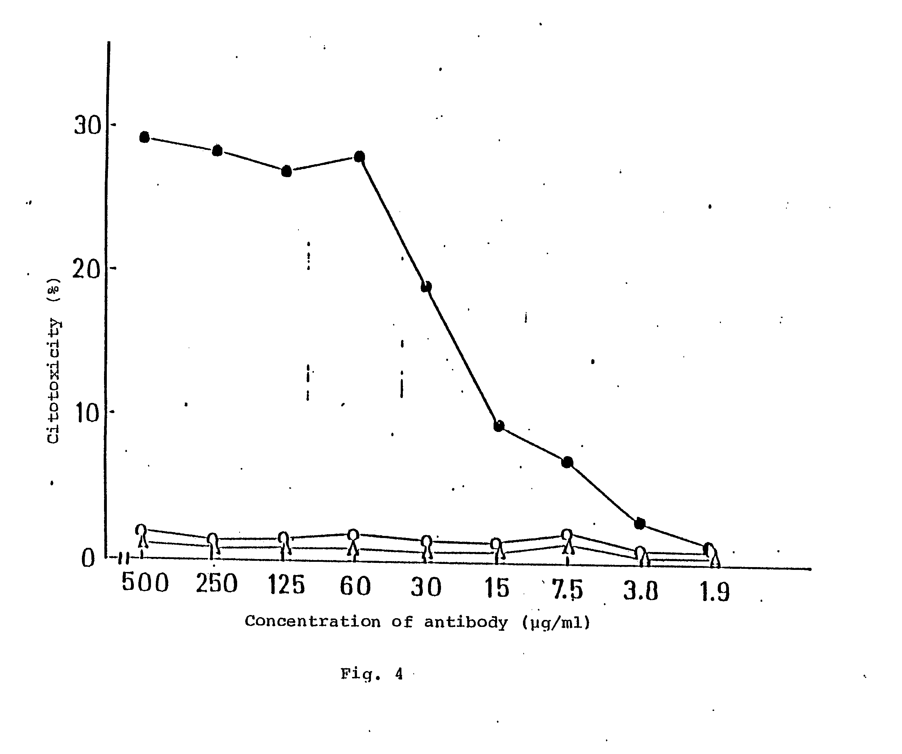

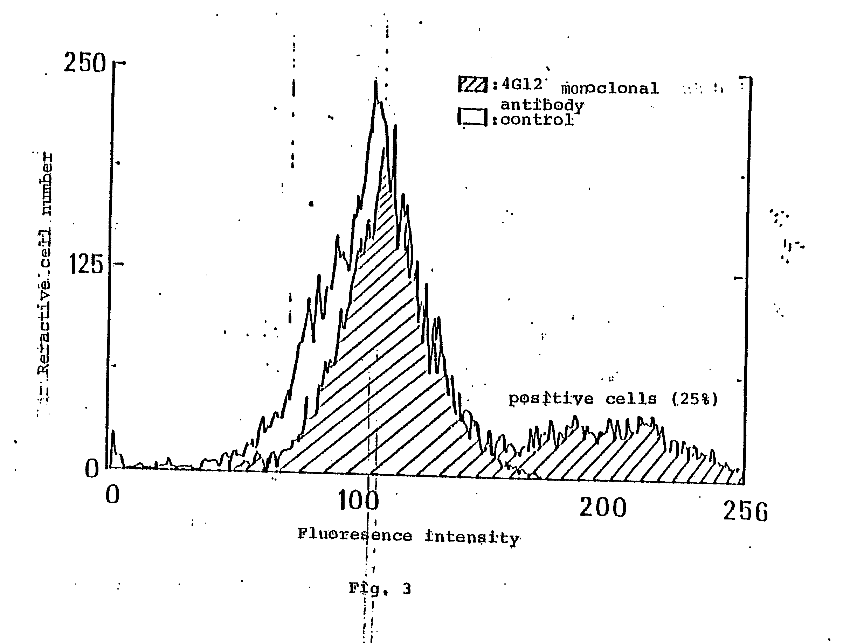

Figure 1 shows the reactivity of 4G12 monoclonal antibody with cell lines determined by the ELISA - (Enzyme linked immunosorbent assay) method, using the cells as targets. In the figure, the following symbols represent the reactivity in each combination of:

O-O : PC10 cells and 4G12 monoclonal antibody

●-● : PC10 cells and the culture supernatant of P3U1 cells

□-□ : PC14 cells and 4G12 monoclonal antibody

■-■ : PC14 cells and the culture supernatant of P3U1 cells

Δ-Δ : fibroblast cells and 4G12 monoclonal antibody

▲-▲ : fibroblast cells and the culture supernatant of P3U1 cells

Figure 2 shows SDS-polyacrylamide gel electrophoresis of an antigen from the PC10 cell surface which reacts with 4G12 monoclonal antibody.

Figure 3 shows an immunofluoresence staining of PC10 cells using 4G12 monoclonal antibody - (FACS analysis).

Figure 4 shows cytotoxicity of 4G12 monoclonal antibody. In the figure, the following symbols show the cytotoxicity in combination of:

●-● : PC10 cells, 4G12 monoclonal antibody and guinea pig complement

O-O : PC10 cells, human IgM antibody and guinea pig complement

Δ-Δ : PC10 cells and 4G12 monoclonal antibody.

Figure 5 shows the stability of the 4G12 monoclonal antibody-producing hybridoma. In the figure, symbol A shows 4G12 monoclonal antibody-producing cells screened after cell fusion, symbol B is cells after first cloning, symbol C is cells after second cloning, and symbols D through H show stability of the 4G12 monoclonal antibody-producing cells obtained in C after a different number of days in culture.

Marks ■■■ and ▭ show respectively the reactivity of 4G12 monoclonal antibody with PC10 cells and the reactivity of the P3U1 cell culture supernatant and PC10 cells measured by absorbance (405 nm) by the ELISA method using cells as target. Mark ▨ shows theantibody concentration produced in the culture supernatant in the hybridoma of the present invention.

Disclosure of the Invention

The human monoclonal antibody of this invention has the following properties:

(i) It is reactive over a wide range, e.g., with human lung cancer cell lines and excized human lung cancer tissues of various histological types including 100% (29/29) squamous lung cancer, 70% (14/20) adeno lung cancer and 37.5% (3/8) large cell lung carcinoma (excluding lung small cell carcinoma), but scarecely reacts with normal human fibroblast and normal tissue.

(ii) It reacts with various other malignant tumors (e.g. thyroid cancer, breast cancer, esophagus cancer, stomach cancer and extra hepatic bile duct cancer etc.) but not with benign tumor tissues to the same extent.

(iii) The antibody is of the IgM class and the light chain type is λ ; it has a complement binding ability and shows complement-dependent cytotoxicity.

(iv) It recognizes antigen molecule having a molecular weight of about 200Kd (according to the SDS-PAGE method) on the surface of PC10 cell membrane which is divided into subunits having molecular weights of about 65Kd according to the SDS-PAGE method under reducing conditions; and is an antibody having high reactivity to lung cancer tissue. Further the human-mouse hybridoma producing the same is stable over long periods of time.

According to the present invention, the hybridoma producing the human monoclonal antibody was established by cell fusion of lymph node cells from patient with human lung squamous cancer and mouse myeloma cells in the presence of a cell fusion agent, culturing in a selection medium and cloning hybridomas producing the desired antibody in the obtained fused cells. A hybridoma which is stable over long periods of time and produces an antibody capable of widely reacting with human lung cancers can be obtained by using lung cancer cell lines especially having different tissue type as target cells or an additional operation for selecting λ-light chain upon cloning. By further culturing this hybridoma, the human monoclonal antibody of the present invention can be produced.

These steps will be described in more detail.

(I) Production of Hybridoma

a) Antibody-producing cells

The antibody-producing cells which are used for cell fusion according to the present invention are human lymphocytes from lung squamous cancer patients. Human lymphocyte populations of lung cancer patients are expected to contain cells that can produce antibodies capable of reacting with human lung cancer cells. Therefore, lymphocytes from lymph nodes from such a patient can be used.

The antibody-producing cells that can be used in the present invention are lymphocytes of lymph nodes removed from a squamous cell carcinoma patient. When the removed lymph nodes are too close to the tumor, immune tolerance to the tumor occurs; conversely when they are too remote, the sensitized state of lymphocytes is insufficient. Therefore, it is appropriate that mediastinal lymph nodes which are most suitably sensitized be selected for the fusion to establish anti-lung cancer antibody producing hybridomas.

b) Myeloma cell

As the myeloma cells, conventional mouse-derived established cell lines are used. Examples include P3-X63-Ag8-U1 (P3U1), SP2/0-Ag14 (SP-2), P3-X63-Ag8-6.5.3 (X63.6.5.3), P3-X63-Ag8 (X63), P3-NS-1-1-Ag4-1 (NS-1), MPC11-45.6.TGI.7 (MPC-11), S194/5XXO.BU.1 (S194), etc. These cell lines are resistant to 8-azaguanine (AZG) or 6-thioguanine and can be selected in hypoxanthine-aminopterin thymidine (HAT) medium; among them, particularly preferred myeloma cell lines are non-immunoglobulin-secreting myeloma cells. These myeloma cells are subcultured in normal media from which 8-azaguanine or 6-thioguanine is removed about 2 weeks before the fusion. On the day of the fusion, cells in the exponential growth phase are used. The normal media can be any of the media in which animal culture cells can grow, for example, RPMI 1640 containing 10% fetal calf serum (FCS) or Dulbecco's modified medium (DMEM).

c) Method of Cell Fusion

The cell fusion is carried out by mixing the aforementioned human lymph node cells and mouse myeloma cells in a cell number ratio of 2.5 to 5:1 and treating the mixture in the presence of a fusion agent. As the fusion agent, one can use polyethylene glycol (PEG, molecular weight of 1000 to 6000), polyvinyl alcohol, Sendai virus (HVJ) and its inactivated products, etc. Further, electric fusion can also be used. However, it is best to use PEG having a molecular weight of 1500. The fusion is carried out as follows: both cells are brought into close contact by centrifugation. Then, the cells are mixed to fuse membranes over several minutes, while gently adding the fusion agent. Thereafter, the fusion mixture is gradually diluted in a serum-free medium and the supernatant is removed by centrifugation. After the cells are suspended in 10% FCS-containing RPMI 1640 medium, the suspension is dispensed into 96-well flat bottom plates (about 2.5 to $5 \times 10^5$ cells/well), followed by incubation.

d) Selection of Fused Cells

From the day after the incubation, half the medium in the well is replaced daily with HAT medium supplemented with hypoxanthine, aminopterin and thymidine or its modified medium to stepwise increase the aminopterin concentration in the medium. In this selection medium, the myeloma cells and human lymphocytes that failed to fuse are killed but fused cells alone grow.

When extremely small numbers of cells are seeded in the wells, feeder cells may be used to assist the growth of the desired cells. As the feeder cells, thymocytes, peritoneal exudate cells, or spleen cells of healthy animals, etc. can be used. The fused cells selected in HAT medium are stepwise transferred to ordinary media, due to the possibility that aminopterin might remain in the medium.

e) Detection of the desired antibody-producing hybridomas

From the fused cells obtained as described above, the desired antibody-producing hybridomas are detected. For the detection, enzymeimmunoassay (EIA) method, radioimmunoassay (RIA) method or fluorescence-activated cell sorter (FACS), etc. can be utilized. In the present invention, in order to accurately detect the desired antibody-producing hybridomas, an ELISA method using cells as targets is used. According to this method, established human lung cancer cell lines are previously cultured and the cell suspension is separately dispensed in a 96-well microplate at a concentration of, e.g., $1 \times 10^5$ cells/well upon assay, without immobilization with glutaraldehyde. After centrifugation, the supernatant is taken out from the plate and about 20 $\mu$l of the culture supernatant of hybridoma is added. After reacting at room temperature for an hour, washing and centrifugation are repeated and followed by incubation with alkaline phosphatase-conjugated anti-human antibody. After washing and centrifugation, an enzyme substrate (for example, disodium p-nitrophenylphosphate) is used to develop a color, whereby its absorbance is measured with a microplate photometer.

In this case, by using a lung cancer cell line derived from the same tissue type (squamous cell carcinoma in the present invention) as that.of the lung cancer of the patient from which the lymphocytes used for the cell fusion have been removed, and, at the same time, a lung cancer cell line derived from a different tissue type as a target, the antibodies capable of widely reacting with human lung cancer can be detected.

f) Identification of class and subclass of immunoglobulin

Class and subclass of immunoglobulin is determined by the ELISA method or Ouchterlony's method (Menekigaku Jikken Nyumon (Guide to Immunological Experiment), Seibutsugaku Jikkenho (Method for Biological Experiment), 15, 75, 1982).

g) Establishment of a stable hybridoma having a high antibody productivity

The obtained hybridoma should be cloned as early as possible. For cloning, the limiting dilution method and the soft agar method are ordinarily used. The limiting dilution is preferably used, in which case SD rat thymocytes, etc. can be added as feeder cells, if necessary, for purposes of assisting proliferation of the hybridoma. Further, in this case, clones which continue to produce monoclonal antibodies stably over long periods of time can also be selected based on the light-chain class determined by the method of f) described above. For instance, in the human-mouse hybridoma, the second human chromosome, which contains a gene of $x$-light chain, is easily lost, so that antibody production is unstable and it is difficult to expect a stable cell line over long periods of time..It is preferred that a $\lambda$-light chain antibody-producing cell line is first selected as described above, and the ELISA method is then conducted again using a lung cancer cell line as a target to select clones producing antibodies capable of specifically reacting with human lung cancer in large quantities.

h) Storage of hybridoma

The hybridoma and clone (subline) are suspended in freezing medium (FCS containing 5 to 10% dimethylsulfoxide (DMSO) or glycerol) at concentrations of $10^6$ to $10^7$ cells/ml, broken down to suitable amounts and frozen.

(II) Production of human monoclonal antibody

The hybridoma which secretes the desired antibody in the foregoing steps a) through g) can provide large quantities of antibodies by culture in vitro or in vivo. For in vitro culture, spinner culture or static culture can be used, while for in vivo growth, the hybridoma is transplanted into an animal capable of allowing growth of the transplanted tumor cells, e.g. nude mouse or nude rat. The antibodies are then obtained from the ascites or blood.

Purification of the antibodies obtained from the culture solution, ascites, etc. can be performed through combinations of the methods generally used for separation of proteins, such as salting out, centifugal separation, dialysis, chromatography, ultrafiltration and freeze drying.

The antibodies of the present invention can be stored in various buffer solutions such as phosphate buffered saline (PBS) (containing an azide), which gives a generally stable antibody solution for further use.

(III) Reactivity with vital tissue

In order to examine the specificity of the produced monoclonal antibody in more detail, freshly frozen slices were prepared both from tumor specimens surgically removed from patients with lung cancer and from normal tissue upon autopsy and were immobilized for 30 minutes at -20°C in an immobilizing solution of 90% ethanol, 5% acetic acid and 5% distilled water. The prepared monoclonal antibody and FITC - (fluorescent isothiocyanate) or peroxidase conjugated anti-human antibody were used as primary and secondary antibodies, respectively. Tissue staining was performed using the indirect immunofluorescent or immunoperoxidase technique and the tissue examined microscopically. In the present invention, a reaction with tumor tissue was determined as being positive when stained and negative when not stained. Further, the normal tissue was determined by comparison with the positively reactive tumor tissue used as a control, as being weakly positive (±) when the staining was obviously weak, positive (+) when it was stained to almost the same degree as the tumor tissue, and negative (-) when not stained.

(IV) Utility of 4G12 monoclonal antibody

The antibody of the present invention can be labeled, e.g. with a radioactive or fluorescent label, and used to identify lung cancer cells. Furthermore, the antibody can be used therapeutically to treat patients suffering from lung cancer; the antibody can be administered to the patient alone or can be administered coupled to a cytotoxic agent.

The antibody can be used to detect lung cancer cells, or the antigen of lung cancer in plasma and other body fluids, both in vivo and in vitro. For in vivo detection of lung cancer cells, the antibody can be radiolabeled by using conventional techniques and, in conjunction with conventional in vivo imaging techniques used to detect labeled immune complexes. The labeled antibody can be administered to a patient and used to identify tumor sites in the patient. The antibody can also be used to measure the amount of the antigen of lung cancer in a clinical sample for early detection of the disease or for monitoring tumor mass.

The monoclonal antibody produced according to the method of the present invention has the following properties:

(1) It reacts with a human lung squamous cell carcinoma cell line (PC10) and a human lung adenocarcinoma cell line (PC14). It does not react with human adult skin-derived fibroblast cells (Figure 1).

(2) On sections of tumor tissue removed from lung cancer patients, it reacts with lung squamous cell carcinoma, lung adenocarcinoma and lung large cell carcinoma in rates of 29/29, 14/20 and 3/8 respectively. It does not react with lung small cell cancer and other mediastinal tumors (Table 1).

(3) It never reacts with normal tissues except for trachea (gland cells), bronchi (gland cells) and kidney (renal tubular cells) (Table 2), nor does it react with benign tumors (Table 4).

(4) It reacts with nonpulmonary tumor tissues such as thyroid cancer, breast cancer, esophagus cancer, stomach cancer, and extra hepatic bile duct cancer in rates of 1/5, 2/5, 3/5, 2/5 and 1/5 respectively (Table 3).

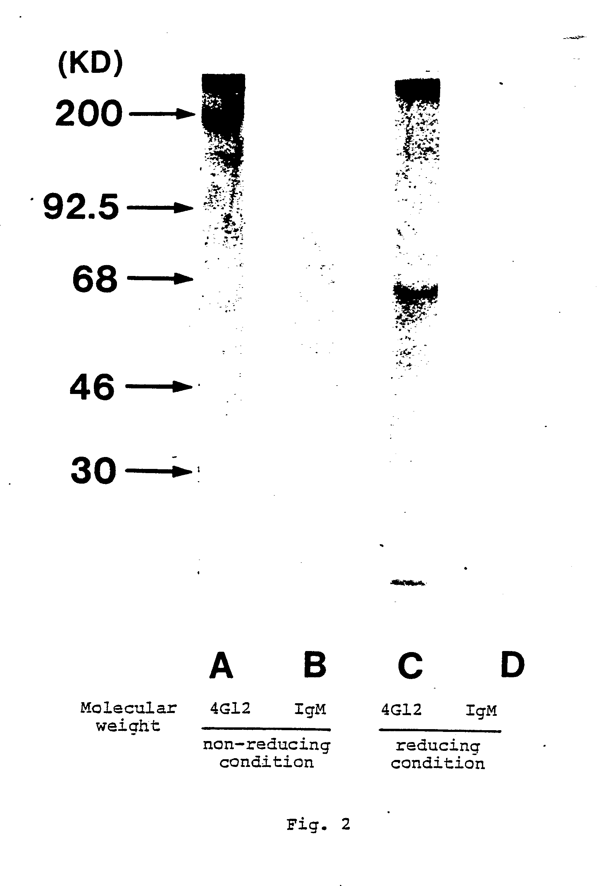

(5) It recognizes an antigen molecule having a molecular weight of about 200Kd according to the SDS-PAGE method in the soluble fraction on the surface of cellular membranes of PC10 cells. The antigen is divided into subunits components having molecular weights of about 65 Kd according to the SDS-PAGE method under reducing conditions (Figure 2).

(6) The class of the antibody is IgM and the light chain type is λ.

(7) It has a complement-binding ability and induces complement-dependent cytotoxicity (Figure 4), the monoclonal antibody has a high reactivity with lung cancers and reacts with other cancers having a common antigen with human lung cancer but rarely reacts with normal human tissues and benign tumors.

Furthermore, the antibody of the present invention uses λ-light chain so that the human-mouse hybridoma produced is stable over long periods of time.

The obtained monoclonal antibody, having a high reactivity with lung cancer tissues, can be used e.g. as an agent for investigations on lung cancers or as a diagnostic and therapeutic agent, such as in serological diagnosis, radioimaging of tumor using labeled antibody or administration of the antibody for an anti-tumor effect. In addition to basic investigative applications such as analysis of tumor antigen.

Hereafter the present invention will be described concretely; it is not deemed to be limited to these examples.

Example 1

(1) Preparation of lymphocytes in lymph node belonging to the lung squamous cell carcinoma patient

A lymph node belonging to a lung squamous cell carcinoma patient were aseptically removed. After superfluous fat tissue was taken out in RPMI 1640 medium, the lymph node was passed through a steel mesh to separate it into single lymphocytes. Washing and centrifugation were repeated in RPMI 1640 medium to remove carbon particles, etc. contained in the lung cancer lymph node. Finally, a single cell suspension in RPMI 1640 medium was made

(2) Cell fusion

A suspension of RPMI 1640 medium containing $4 \times 10^7$ mouse myeloma cells P3U1 (Curr-Top. Microbiol. Immunol., 81, 1-7, 1978) and the suspension of RPMI 1640 medium containing $2 \times 10^8$ lymph node cells prepared in (1) above were mixed in a 50 ml tube followed by centrifugation at room temperature at 400 x g for 5 minutes. The supernatant was completely removed and the tube was put in a incubator at 37°C. While gently stirring the cells with a tip of a pipette, 1 ml of warm 50% polyethylene glycol (PEG) solution was added over one minute. After stirring for another minute with the same pipette, 2 ml of RPMI 1640 medium warmed to 37°C was added over 2 minutes with gentle stirring followed by centrifugation at room temperature at 400 x g for 10 minutes. The supernatant was removed. After 40 ml of RPMI 1640 medium containing 10% FCS was added and the mixutre was gently stirred, this cell suspension was dispensed into 96 well flat bottom microplates (0.1 ml/well) followed by incubation at 37°C in a humid 5% $CO_2$ atomosphere.

(3) Selection of fused cell

On the day after initiation of the incubation, 0.1 ml of HAT medium was added to each well of the 96 well flat bottom microplates. On Days 2, 3, 4, 6, 8 and 10, half of the supernatant of each well was removed and 0.1 ml of fresh HAT medium was added. Thereafter the medium exchange was performed with HT - (hypoxantine-thymidine) medium every other day and fused cells were finally cultured in 10% FCS-containing RPMI 1640 medium.

(4) Detection of the desired antibody-producing hybridomas

The supernatants in the wells in which colonies had been formed were tested using the ELISA method. Three kinds of cells, PC10 (lung cancer cell line of human squamous cell carcinoma type), PC14 (human lung adenocarcinoma cell line), and a normal human fibroblast cell line were used as target cells. Target cells were dispensed in a 96 well microplate (ca. $1 \times 10^5$ cell/well) and 20 $\mu l$ of the culture supernatant of each colony was added and allowed to react at room temperature for 60 minutes. After the cells were washed by centrifugation, 20 $\mu l$ of alkaline phosphatase-conjugated rabbit anti-human antibody was added and left at room temperature. After the cells were washed again by centrifugation, a chromophoric substrate and a buffer were added to the cells to form a color, thereby showing the reactivity with the target cells. The degree of color formation was measured as absorbance of 405 nm light with a microplate photometer. A well showing absorbance of more than twice that of the control (the culture supernatant of P3U1) was designated a positive well. Thus, a hybridoma which reacted with the tumor cell lines PC10 and PC14 but did not react with normal human fibroblast cells was obtained.

(5) Reactivity with fresh frozen tissue

With respect to the hybridoma screened by the aforementioned ELISA method using cells as targets, the reactivities with the tumor tissue removed from a lung cancer patient and normal tissue obtained upon autopsy were determined using the indirect fluorescent antibody technique. The activity was measured by the presence or absence of staining. Those that showed a high reactivity with the tumor tissue but did not react with the normal tissue were cloned by limiting dilution. In this case, the cell number of the hybridoma was adjusted to a concentration of 30 cells/20 ml using SD rat thymocytes as feeder cells (feeder layer). The hybridoma was distributed in a 96-well flat bottom microtiter plate (0.2 ml/well). When colonies grew to an appropriate size, their reactivities with the tumor tissue and the normal tissue were again determined by the indirect fluorescent antibody technique. Hybridoma 4G12 was obtained as a stable cell line producing the antibody in large quantities.

### (6) Purification of antibody

For purification, either the culture supernatant obtained by in vitro incubation of the hybridoma or ascites containing the antibody obtained by intraperitoneally administering 1 to 3 x $10^7$ hybridomas to BALB/c nude mouse was purified. A 10-fold concentrate of 1300 ml of culture supernatant (with Aquaside, Calbiochem) or about 10 ml of the ascites collected were centrifugated and each supernatant was put dialysed for 48 hours against 0.001M phosphate buffer (pH 6.0). By this procedure the desired monoclonal antibody could be precipitated. The precipitated monoclonal antibody can be dissolved in a small amount of 3% sodium chloride. The solution was dialyzed against 0.1M phosphate-sodium chloride buffer (pH 7.2).

### (7) Identification of the class and subclass of immunoglobulin

Each class and subclass of immunoglobulin were identified by the ELISA method using antibodies specific to each class and subclass of anti-human immunoglobulin and anti-light chain antibody. As a result, 4G12 monoclonal antibody was identified as being of the IgM isotype with $\lambda$ light chain.

### (8) Property of 4G12 monoclonal antibody

### i) Reactivity with established cell lines

The reactivities with the human lung squamous carcinoma cell line (PC10) and the human lung adenocarcinoma cell line (PC14) and with human adult skin-derived fibroblast cells as a normal cell control were examined by the ELISA method. The results are shown in Figure 1. It is evident that 4G12 monoclonal antibody shows a remarkable reactivity with human lung cancer cell lines (PC10 and PC14).

### ii) Reactivity with tumor and normal tissues

The reactivity of 4G12 monoclonal antibody with each tissue was examined by the indirect immunofluorescence or immunoperoxidase technique. The results are shown in Tables 1 through 4. It is evident that the human monoclonal antibody of the present invention reacts strongly with human lung cancers and can be reactive with nonpulmonary tumor tissues such as thyroid cancer, breast cancer, esophagus cancer, stomach cancer, extra hepatic bile duct cancer, etc. but rarely reacts with normal human tissues and benign tumors.

### iii) Accumulation of $^{125}$I-labeled 4G12 monoclonal antibody in human lung cancer xenograft.

Accumulation of radiolabeled 4G12 monoclonal antibody was investigated by autoradiography. The technique of whole body autoradiography was used to study the local accumulation of radioactive 4G12 monoclonal antibody in sections of tumor-bearing nude mice. Seven days after the $^{125}$I-labeled antibody injection (50 $\mu$Ci: specific activity is 1 $\mu$Ci/$\mu$g), mice were sacrificed, freeze-dried and sliced 40 $\mu$m thick with an autocryotome. Sections were air-dried for 24-48 hours at 4°C and then exposed to an X-ray film at room temperature for 2-4 days.

The radioactivity was specifically accumulated in the tumor region. The radioactivity of 4G12 monoclonal antibody when expresssed as a percent dose/g tissue was much greater in the tumor than in other organs. The ratio of uptake of radioactivity (derived from the organ/muscle ratio) was 42.7 (Table 5).

8

Table 1
Immunohistochemical Reactivity of 4G12 Monoclonal Antibody with Various Lung Tumor Tissues

| | | | Reactivity | | |
|---|---|---|---|---|---|
| | Tumor tissues | No. | Positive samples | No. | Negative samples |
| Primary | Squamous cell carcinoma | 29 | | 0 | |
| Lung cancer | Adenocarcinoma | 14 | | 6 | |
| | Adenosquamous cell carcinoma | 1 | | 0 | |
| | Large cell carcinoma | 3 | | 5 | |
| | Small cell carcinoma | 0 | | 8 | |
| Metastatic | Thyroid cancer* | 1 | | 0 | |
| Lung cancer | Colon cancer* | 1 | | 0 | |
| | Gastric cancer* | 1 | | 0 | |
| | Breast cancer* | 0 | | 2 | |
| | Renal cell carcinoma* | 0 | | 2 | |
| | Chorioepithelioma* | 0 | | 1 | |
| Other Lung | Adenoid cystic carcinoma | 2 | | 0 | |
| Tumors | Carcinoid | 0 | | 4 | |
| | Hamartoma | 0 | | 3 | |
| | Sclerosing hemangioma | 0 | | 3 | |
| Mediastinal | Thymoma | 0 | | 5 | |
| Tumors | Schwannoma | 0 | | 5 | |
| | Intra thoracic goiter | 0 | | 3 | |
| | Dermoid cyst | 0 | | 2 | |

* Primary Tumor

0 232 871

Table 2. Immunohistochemical Reactivity of 4G12 Monoclonal Antibody with Normal Tissues

| Normal Tissues | Reactivity | Normal Tissue | Reactivity |
|---|---|---|---|
| Respiratory Tract Organ: | | Endocrinium Organ: | |
| Trachea | + (gland cells) | Pancreas | — |
| Bronchus | + (gland cells) | Thyroid gland | — |
| Peripheral lung tissue | — | Adrenal gland | — |
| Digestive Tract Organ: | | Hematopoietic Organ: | |
| Tongue | — | Erythrocyte | — |
| Esophagus | — | Polymorphonuclear leukocyte | — |
| Stomach | — | Lymphocyte | — |
| Small intestine | — | Bone marrow cells | — |
| Large intestine | — | Spleen | — |
| Liver | — | Lymph node | — |
| Cardio Vasocular System Organ: | | Urinary Tract Organ: | |
| Heart | — | Kidney | ± (tubular cells) |
| Artery | — | Urinary bladder | — |
| Nervous System Organ: | | Reproductive System Organ: | |
| Cerebrum | — | Ovary | — |
| Cerebellum | — | Uterus | — |
| Spinal cord | — (substantia alba or grisea) | Testis | — |
| Peripheral nerve | — | Prostate | — |
| Skin | — | Cross-Striated Muscles | — |
| Mammary Grand | — | | |

0 232 871

Table 3. Immunohistochemical Reactivity of 4G12 Monoclonal Antibody
with Various nonpulmonary Tumor Tissues

| Tumor Tissues | Histology | Reactivity | |
|---|---|---|---|
| | | No. Positive samples | No. Negative samples |
| Thyroid | Adeno | 1* | 4 |
| Breast | Adeno | 2 | 3 |
| Esophagus | Squamous | 3* | 2 |
| Stomach | Adeno | 2 | 3 |
| Colon | Adeno | 0 | 5 |
| Pancreas | Adeno | 0 | 3 |
| Gall bladder | Adeno | 0 | 3 |
| Extra hepatic bile duct | Adeno | 1 | 4 |
| Liver | Hepato cellular | 0 | 3 |

\* Weakly stained

## Table 4. Immunohistochemical reactivity on various benign tumor tissues

| Tumor Tissues | Reactivity[a] | |
| --- | --- | --- |
| | No. Positive samples | No. Negative samples |
| Lipoma | 0 | 2 |
| Hamartoma | 0 | 4 |
| Dermoid cyst | 0 | 2 |
| Intra-thoracic goiter | 0 | 3 |
| Sclerosing hemangioma | 0 | 3 |

[a] Stained by immunoperoxidase technique.

## 0 232 871

Table 5.  Organ distribution of $^{125}$I-labeled 4G12 MoAb

in human lung cancer (PC10) bearing nude mice

| Organ | Organ / Muscle ratio |
| --- | --- |
| Muscle | 1.0[a] |
| Tumor | 42.1 |
| Lung | 1.7 |
| Brain | 0.5 |
| Intestine | 1.8 |
| Liver | 4.8 |
| Bone marrow | 1.9 |
| Heart (Blood) | 6.5 |
| Skin | 5.9 |

[a] Specific radioactivity is 36.9 nCi/g.

iv) Analysis of antigen recognized by 4G12 monoclonal antibody

On human lung squamous cell carcinoma line (PC10), which showed high reactivity with 4G12 monoclonal antibody, an antigen recognized by the antibody should be present. Based on this, 1 x 10$^8$ PC10 cells were labeled with $^{125}$I-Na on the surface of the cellular membrane. Its soluble fraction was then incubated with the purified monoclonal antibody overnight at 4°C. There after the antibody-antigen complex was precipitated using protein A-labeled anti-human IgM antibody. The precipitates were analyzed using 10% SDS polyacrylamide gel electrophoresis. As shown in Figure 2 the 4G12 monoclonal antibody recognized an antigen molecule having a molecular weight of about 200Kd.

Further, this antigen was divided into two components each having molecular weights of about 65 Kd when analyzed under reducing conditions.

Furthermore, the tumor tissue which was shown to be strongly positive with 4G12 monoclonal antibody was treated with 5% periodic acid for 30 minutes, after which the reactivity was fully lost. This implies that sugar chains help form its antigenic determinant.

v) Distribution of recognition antigen

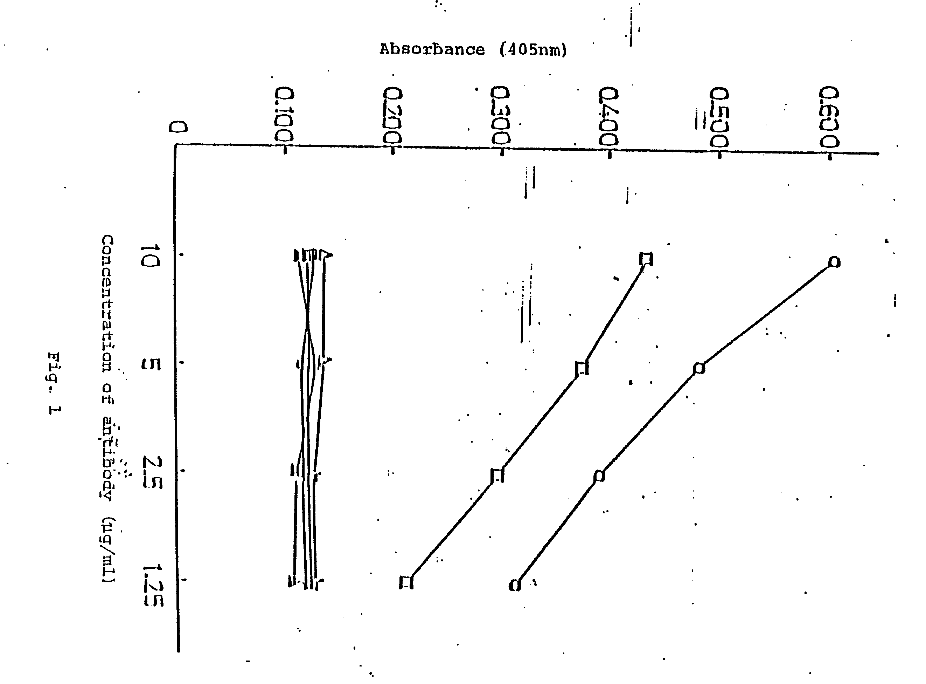

PC10 cells were subjected to membrane staining using 4G12 monoclonal antibody and examined by a flouroscence-activated cell sorter (FACS). According to this method, PC10 cells, 4G12 monoclonal antibody and FITC-conjugated anti-human IgM antibody were used as a target cell, primary antibody and secondary antibody, respectively and the flourescing cells were measured by the cell sorter. The cells were consequently divided into two groups: a non-reactive cell group in which the cells were stained as weakly as the control cells, and the reac tive cell group in which they were strongly stained, as shown in Figure 3, and showed a two-phase pattern. 25% of the PC10 cells were reactive. The cells reactive with the 4G12 monoclonal antibody and the non-reactive cells were independently subjected to sorting and the respective cells were separately incubated. The incubated cells were subjected to membrane staining like-wise. As expected, both the reactive cells and the non-reactive cells showed a two-phase pattern.

Moreover, FACS analysis of lung squamous cell line PC10 demonstrated that the expression of antigen on the cell surface depended on the cells stage in the cell cycle: maximum in $G_2$ and M phases and minimum in S and $G_1$ phases, suggesting that the antigen recognized by 4G12 monoclonal antibody of the present invention is expressed on the surface of the cells in the proliferative or dividing stages rather than in the resting state.

In addition, the staining pattern of 4G12 monoclonal antibody against lung squamous cell carcinoma suggests that the antigen expression is somehow related to tissue differentiation. 4G12 monoclonal antibody reacts strongly and homogeneously with both membrane and cytoplasm of tumor cells of well-differentiated lung cancer tissues, while heterogenously and rather weakly staining poorly-differentiated tissues. Thus, the antigen recognized by 4G12 monoclonal antibody seems to be a kind of differentiation antigen. This also indicates that 4G12 monoclonal antibody is a good probe for biochemical analysis of proliferation and differentiation of lung carcinoma cells.

vi) Cytotoxicity of 4G12 monoclonal antibody

The cytotoxicity of 4G12 monoclonal antibody for PC10 target cells was assayed by adding complement derived from a guinea pig. The maximum cytotoxicity was 27% of all cells, as shown in Figure 4. This suggested that the monoclonal antibody mediated complement-dependent cytotoxicity.

(9) Stability of 4G12 monoclonal antibody-producing hybridoma

Human-mouse heterohybridomas frequently demonstrate deletions of human chromosomes so that they often discontinue the production of antibody at an early stage even though a fused line producing the desired antibody is originally obtained. Accordingly, it is thought to be very difficult to establish a fused line having stability over a long period of time and a high production rate. Therefore, with respect to the hybridoma that secretes 4G12 monoclonal antibody, the antibody concentration produced in the culture supernatant was examined daily from the time of fusion by the ELISA method, and the reactivity of the produced antibody with PC10 cells was measured with the ELISA method using cells as targets. As a result, a line having a high titer could be obtained only by performing cloning twice at an early stage, as shown in Figure 5, though high values were not shown in the cell binding assays during the screening. Since the establishment of the cell line, the hybridoma has secreted the monoclonal antibody very stably without decreasing its productivity. 24 months after the fusion, the antibody concentration after incubation for 2 days showed a value of 10μg/ml. Further, the hybridoma also produced a large amount of human monoclonal antibody (4 mg/ml) in nude mice. Therefore, it can be said that the hybridoma has high productivity of the antibody relative to other human-mouse heterohybridomas.

The aforementioned hybridoma producing anti-lung cancer human monoclonal antibody (4G12) was deposited on March 7, 1986 in Institute for Fermentation, Osaka (IFO) under IFO 50090.

## Claims

(1) Human monoclonal antibody produced by a human-mouse hybridoma which is obtained by cell fusion of lymph node cells from patient with human squamous lung carcinoma and mouse myeloma cells.

(2) The antibody of claim 1, wherein said antibody reacts strongly with human lung cancer (excluding lung small cell carcinoma) and can be reactive with nonpulmonary tumors, but rarely reacts with normal human cell and benign tumors.

(3) The antibody of claim 1, wherein said antibody recognizes antigen molecule having a molecular weight about 200Kd under non-reducing conditions and about 65Kd under reducing conditions on the surface of PC10 cell.

(4) The antibody of claim 1, wherein said antibody is of the IgM isotype with λ light chain.

(5) The antibody of claim 1, wherein said antibody is named 4G12 monoclonal antibody produced by the hybridoma given IFO Accession No. 50090.

(6) The antibody of claim 1, wherein said antibody is labeled with a detectable label.

(7) The antibody of claim 6, wherein said antibody is radiolabeled or fluorescently labeled.

(8) A human-mouse hybridoma for producing human monoclonal antibody which is obtained by cell fusion of lumph node cells from patients with human squamous lung carcinoma with mouse myeloma cells.

(9) The hybridoma of claim 8, wherein said hybridoma is given IFO Accession No. 50090.

(10) The use of the antibody of claim 1 in an immunoassay for the detection of human lung cancer.

Fig. 5

Fig. 4

Fig. 3

:4G12 monoclonal antibody
:control

positive cells (25%)

Fluorescence intensity

Relative cell number

250

125

0

0    100    200    256

Fig. 2

Fig. 1